(19) Europäisches Patentamt
European Patent Office
Office européen des brevets

(11) **EP 1 970 677 B1**

(12) **EUROPÄISCHE PATENTSCHRIFT**

(45) Veröffentlichungstag und Bekanntmachung des Hinweises auf die Patenterteilung:
**07.10.2009 Patentblatt 2009/41**

(51) Int Cl.:
*G01F 11/00* (2006.01)         *A61M 5/168* (2006.01)
*A61M 5/142* (2006.01)

(21) Anmeldenummer: **07104240.2**

(22) Anmeldetag: **15.03.2007**

(54) **Infusionssystem mit einer Dosiervorrichtung**

Intravenous infusion system with dosing device

Système d'infusion avec dispositif de dosage

(84) Benannte Vertragsstaaten:
**AT BE BG CH CY CZ DE DK EE ES FI FR GB GR HU IE IS IT LI LT LU LV MC MT NL PL PT RO SE SI SK TR**

(43) Veröffentlichungstag der Anmeldung:
**17.09.2008 Patentblatt 2008/38**

(73) Patentinhaber:
• **F. Hoffmann-La Roche AG**
**4070 Basel (CH)**
Benannte Vertragsstaaten:
**AT BE BG CH CY CZ DK EE ES FI FR GB GR HU IE IS IT LI LT LU LV MC MT NL PL PT RO SE SI SK TR**
• **Roche Diagnostics GmbH**
**68305 Mannheim (DE)**
Benannte Vertragsstaaten:
**DE**

(72) Erfinder:
• **Haueter, Ulrich**
**3506 Grosshöchstetten (CH)**
• **Kühni, Florian**
**3400 Burgdorf (CH)**

(74) Vertreter: **Schwabe - Sandmair - Marx**
**Patentanwälte**
**Stuntzstrasse 16**
**81677 München (DE)**

(56) Entgegenhaltungen:
**EP-A- 0 980 690          EP-A- 1 486 218**
**EP-A- 1 754 505          WO-A-20/07000064**
**DE-A1- 3 832 028          US-A- 3 631 654**
**US-A- 5 807 321**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung bezieht sich auf ein Infusionssystem mit einer Dosier- und Fordervorrichtung und insbesondere auf eine Vorrichtung zum Dosieren einer mittels eines Infusions-Sets an einen Patienten abzugebende Substanz.

Aus der CH 688 224 A5 ist eine implantierbare Vorrichtung für die dosierte Abgabe pharmazeutischer Flüssigkeiten im menschlichen oder tierischen Körper bekannt, wobei die implantierbare Einheit eine kurvengesteuerte, ventillose Axialkolbenpumpe mit einem drehantreibbaren und axial verschiebbaren Kolben, ein mit der Saugseite der Pumpe verbundenes Flüssigkeitsreservoir und einen steuerbaren, mit dem Kolben verbundenen Drehantrieb aufweist. Dabei ist an einem unteren Ende eines Zylinders je eine Saugöffnung und eine Drucköffnung angeordnet, wobei die beiden Öffnungen sich diametral gegenüberliegen und gleichachsig verlaufen.

Die US 6,010,485 offenbart einen Arbeitszylinder mit einem ähnlichen Aufbau wie in der CH 688 224 A5.

Die US 6,749,587 B2 offenbart ein modulares Infusionsgerät mit einem Messabschnitt, welcher direkt den Fluidfluss zwischen einem Reservoir und einer Kanüle steuern kann. Wenn das Reservoir bei Umgebungsdruck gehalten wird, kann der Messabschnitfi einen Peristaltik-Mechanismus, eine Verdrängerpumpe oder eine andere Pumpvorrichtung aufweisen.

Aus der US 4,643,723 ist eine Vorrichtung zur Verabreichung von Insulin an einen Patienten bekannt, wobei in einer Pumpkammer ein Kolben angeordnet ist, eine Kanüle mit der Pumpkammer verbunden ist und ein Kolbenstab mit dem Kolben verbunden ist. Wenn der Kolben zurückgezogen wird, wird das Reservoir mit der Pumpkammer verbunden, um die Pumpkammer aufzufüllen und wenn der Kolben nach vorne bewegt wird, wird mittels eines Ventils der Durchgang vom Reservoir zur Pumpkammer geschlossen, so dass die Substanz über eine Kanüle verabreicht werden kann.

Aus der WO 93/04714 und der korrespondierenden EP 0 600 948 B1 ist ein Fluidmesselement für ein implantierbares Verabreichungssystem bekannt, welches zwischen einer unter Druck gesetzten Fluidquelle und einer Auslassöffnung gekoppelt ist, um diskrete Flussimpulse mit einer vorgegebenen Rate zur Verfügung zu stellen.

Die US 2004/0069044 A1 offenbart eine Vorrichtung zum Messen eines Volumens eines Arzneimittels. Die Vorrichtung weist eine erste Kammer, welche das flüssige Arzneimittel enthält, eine Messkammer, die in Fluidverbindung mit der ersten Kammer ist und eine Messanordnung auf.

Die US 5,207,666 offenbart ein Fluidmessgerät für implantierbare Arzneiverabreichungssysteme, welches zwischen einer unter Druck gesetzten Fluidquelle und einer Auslassöffnung angeordnet werden kann, um diskrete Flussimpulse mit einer vorgegebenen Rate zur Verfügung zu stellen.

Aus der US 2005/0159708 A1 ist eine Infusionspumpe zur dosierten Verabreichung einer Flüssigkeit bekannt, wobei ein Kolben permanent mittels einer Feder mit Druck beaufschlagt wird und auf einen Medikamentenbehälter einwirkt, der durch eine Abgabeöffnung die Substanz in Richtung auf ein Steuerventil zum Dosieren abgibt.

Die EP 1 633 417 B1 offenbart ein Abgabegerät mit einer Aufbewahrungskammer und einer Injektionskammer, die über eine Fluidverbindung gekoppelt sind und in welchen jeweils Tauchkolben angeordnet sind.

Aus der US 5,807,321 ist ein System zum elektronischen Überwachen der Verabreichung eines Kontrastmediums bekannt, wobei ein Fluidzylinder beweglich in einer zylinderförmigen Kammer gelagert ist. Durch Drehung des Fluidzylinders, welcher Ventilöffnungspaare aufweist, die mit korrespondierenden Öffnungspaaren des Gehäuses der zylinderförmigen Kammer zusammenwirken, können unterschiedliche Flussrichtungen realisiert werden. Ein innerhalb des Fluidzylinders frei bewegbarer Fluidverdrängungsindikator wird in der Fluidkammer des Fluidzylinders bewegt, wenn ein Fluiddruck an dem ersten Ende oder dem zweiten Ende angelegt wird.

Aus der WO 2007/000064 A1 ist eine Dosiervorrichtung bekannt, wobei ein Kolben entgegen der Federkraft einer Feder durch Zuführen einer unter Druck stehenden Flüssigkeit verschoben wird, bis er formschlüssig an einer Stufe des Verdrängungskanals anschlägt, so dass bei dieser Kolbenposition ein genau definiertes maximales Volumen durch den dann entstehenden ersten Verdrängungsraum gebildet wird.

Aus der EP 0 980 690 A2 ist eine Vorrichtung für eine abgemessene Fluidzufuhr bekannt, wobei ein Kolben durch ein unter Druck gesetztes Fluid zwischen zwei Positionen hin- und hergeschoben wird.

Aus der EP 1 754 505 A1 ist ein Aufsatz für eine Spritze bekannt, um sicherzustellen, dass nach dem Befüllen keine Luftblasen in der Spritze verbleiben. Dabei weist die Vorrichtung zwei Anschlüsse auf.

Es ist eine Aufgabe der vorliegenden Erfindung eine Infusionssystem mit einer Dosiervorrichtung vorzuschlagen, welche ein einfaches und genaues Dosieren einer abzugebenden Substanz ermöglicht.

Diese Aufgabe wird durch eine Dosiervorrichtung gemäß Anspruch 1 gelöst. Vorteilhafte Ausführungsformen ergeben sich aus den abhängigen Ansprüchen.

Eine Dosiervorrichtung für ein Infusionssystem weist eine Dosiereinheit mit einem veränderbaren Volumen in Gestalt eines Zylinders mit einem sich in dem Zylinder bewegenden und das Volumen vergrößernden oder verkleinernden Kolben auf. Die Dosiervorrichtung hat einen Zufuhr- und einen Abgabeanschluss, welcher vorzugsweise durch einen einzigen Anschluss, wie zum Beispiel eine einzige Öffnung am Zylinder gebildet wird und ist ansonsten vorzugsweise geschlossen. Durch die Zufuhr- und Abgabeöffnung kann das veränderbare Volumen der Dosiereinheit mit der abzugebenden Substanz zum Beispiel aus einem Vorratsbehälter, wie zum Beispiel einer Ampulle, gefüllt werden, wenn sich das Volumen der Dosiereinheit vergrößert. Wird das veränderbare Volumen der gefüllten Dosiereinheit wieder verkleinert, kann durch die Abgabeöffnung die dosiert abzugebende Substanz wieder ausgegeben werden. Bevorzugt ist die Dosiervorrichtung so ausgebildet, dass der Zufuhr- und Abgabeanschluss, also zum Beispiel die einzige Öffnung der Dosiereinheit, bevorzugt alternierend mit einem Vorratsbehälter, wie zum Beispiel einer Ampulle, und einer Abgabe- oder Verabreichungseinheit, wie zum Beispiel einem Infusions-Set, verbunden werden kann.

Erfindungsgemäß kann somit ein Leck oder ein unbeabsichtigtes und unkontrolliertes Durchtreten einer Substanz aus einem Reservoir zu einer Abgabeeinheit, wie einem Infusions-Set, verhindert werden, da die Dosiereinheit entweder nur mit dem Reservoir oder nur mit der Verabreichungsvorrichtung gekoppelt ist, Das Reservoir ist somit von der Verabreichungseinheit vollständig entkoppelt, also das Reservoir ist vorteilhaft nie direkt mit dem Ausgang verbunden.

Der Zufuhr- und Abgabeanschluss kann auch durch mehrere Öffnungen gebildet werden, welche dann immer so mit einem externen Anschluss verbunden sein sollten, dass die Dosiereinheit durch alle oder einen Teil der Öffnungen entweder nur befüllt oder nur entleert wird, also ein gleichzeitiges Aufnehmen oder Abgeben einer Substanz nicht möglich ist. Das oder die gleichzeitig als Einlass und Auslass wirkenden Verbindungen oder Zugänge oder Löcher der Dosiereinheit ist beziehungsweise sind so koppelbar, dass sie entweder mit einem Reservoir gekoppelt werden können und somit als Einlass für die Dosiereinheit wirken, oder mit einer Verabreichungsvorrichtung, wie zum Beispiel einem Infusionsgerät, gekoppelt werden können, und so als Auslass für die Dosiereinheit wirken. Vorzugsweise sind die Anschlüsse der Dosiereinheit verschlossen, wenn keine Substanz aufgenommen oder abgegeben wird.

Es ist auch möglich in einer Ruheposition zwischen einer Abgabe oder Aufnahme einer Substanz die Öffnungen aktiv z.B. durch ein drehbares oder verschiebbares Dichtelement zu verschließen.

Die Dosiereinheit ist drehbar um je nach Drehstellung mit einem von zwei oder mehr externen Anschlüssen zur Befüllung der Dosiereinheit und zur Abgabe und Weiterleitung der von der Dosiereinheit abgegebenen Substanz verbunden zu werden. Ebenso können auch einer oder mehrere der externen Anschlüsse verschiebbar oder drehbar an der Dosiereinheit vorgesehen sein, so dass die Anschlüsse zum Beispiel abwechselnd mit der Zufuhr- und Abgabeöffnung der Dosiereinheit verbunden werden. Somit kann eine Ventilwirkung durch eine Art Umschaltventil erhalten werden. Nicht erfindungsgemäß kann die Dosiereinheit auch verschiebbar sein.

Es ist ein Motor vorgesehen, mit welchem die Dosiereinheit gedreht oder der Zufuhr- und Abgabeanschluss der Dosiereinheit bewegt (zum Beispiel gedreht oder verschoben) werden kann, wobei der Motor alternativ oder ergänzend auch zur Bewegung, also zum Beispiel zur Verschiebung oder Drehung, der externen Zufuhr- und Abgabeanschlüsse verwendet werden kann, um diese abwechselnd mit der Zufular- und Abgabeöffnung der Dosiereinheit zu verbinden. Ebenso wird der oder ein anderer Motor zur Vergrößerung oder Verkleinerung des Volumens durch Bewegung des Kolbens verwendet . Vorzugsweise ist der Kolben des Zylinders so ausgebildet, dass er genau zu einer vorgegebenen Position innerhalb des Zylinders bewegt werden kann, also zum Beispiel exakt zu einer vordefinierten maximalen Auszugsstellung des Kolbens, welche sich vorteilhaft noch innerhalb des Zylinders befindet. Somit kann anders als bei bekannten so genannten "single stroke" Verfahren auch nur ein Teil des veränderbaren (Zylinder-)Volumens zur Dosierung verwendet werden.

Die Verbindungsstellen oder Verbindungsleitungen, welche zu dem Zufuhr- oder Abgabeanschluss der Dosiereinheit führen, weisen vorteilhaft jeweils mindestens ein Ventil auf, wobei auch nur ein einziges Ventil entweder in der Zufuhr- oder in der Abgabeleitung oder in dem Zufuhr- und Abgabeanschluss vorgesehen sein kann, Ein solches Ventil kann in einer oder beiden der Leitungen zum Beispiel als Rückschlagventil sicherstellen, dass eine dosiert abzugebende Substanz nur in eine Richtung gefördert wird, wobei der Rückfluss der Substanz in die Gegenrichtung unterbunden oder verhindert wird.

Das oder die verwendeten Ventile am oder in den Zu- und Ableitungen und/oder an oder in der Dosiereinheit können Rückschlagventile sein, welche einen Fluss einer Substanz oder einer Flüssigkeit nur in eine Richtung ermöglichen oder können auch als Überdruckventile ausgebildet sein, welche einen Durchgang eines Materials oder einer Flüssigkeit erst nach Anliegen eines Mindestdruckes ermöglichen.

Der Vorratsbehälter, welcher die nach Durchlauf durch die Dosiervorrichtung dosiert abzugebende Substanz enthält, kann druckbeaufschlagt oder drucklos sein. Beispielsweise kann der Vorratsbehälter eine Ampulle sein, in welcher ein druckbeaufschlagter Stopfen auf die abzugebende Substanz einwirkt, so dass die abzugebende Substanz nur noch dosiert werden muss und keine weitere Energie für die eigentliche Substanzabgabe aus der Ampulle bzw, das Aufdosieren oder Umfüllen in den Mikrodosierzylinder benötigt wird. Die Druckbeaufschlagung des Stopfens kann beispielsweise mittels einer Feder oder eines unter Überdruck stehenden Gases erfolgen. Ebenso kann der Vorratsbehälter einen elastischen Bereich aufweisen oder vollständig aus einem elastischen Material gebildet sein, wie zum Beispiel ein Beutel, welcher mit der abzugebenden Substanz gefüllt ist. Auf den elastischen Bereich oder Beutel kann zum Beispiel mittels einer Feder eine Kraft oder ein Druck einwirken, um die in dem Vorratsbehälter enthaltene Substanz zu verdrängen und abzugeben, wenn dies durch die dem Vorratsbehälter nachgeschaltete Dosiervorrichtung ermöglicht wird.

Vorzugsweise ist ein Vorratsbehälter oder Reservoir so ausgelegt oder konstruiert, dass bei einem Ausgang oder einer Abgabeöffnung des Reservoirs ein positiver Druck anliegt, was zum Beispiel durch mit Kraft oder Druck beaufschlagte Behälter oder Beutel, oder durch einen aktiven Antriebsmechanismus, welcher auf ein Verdrängungselement des Reservoirs oder das Reservoir selbst einwirkt, erreicht werden kann,

Vorzugsweise weist die Dosiervorrichtung mindestens einen Sensor zur Überprüfung der Funktionsfähigkeit oder zur Feststellung einer Fehlfunktion auf, wie zum Beispiel einen Leck-Sensor, galvanischen oder Leitwertsensor, einen Blasen-Sensor, einen Druck-Sensor oder einen Kraftsensor, welche zum Beispiel feststellen können, ob eine Substanz oder Flüssigkeit aus der Dosiervorrichtung ausgetreten ist, ob zum Beispiel Blasen in der abzugebenden Substanz enthalten sind, oder ob zum Beispiel eine Okklusion vorliegt. Dieser mindestens eine Sensor kann mit einer Warn- oder Alarmanzeige oder einer Steuerung der Dosiervorrichtung verbunden sein, um die Dosiervorrichtung zum Beispiel abzuschalten, wenn eine Fehlfunktion festgestellt wird oder um im Falle einer festgestellten Störung der Dosiervorrichtung ein zum Beispiel optisches oder akustisches Alarmsignal auszugeben.

Vorteilhaft wird ein gas- oder luftdurchlässiges Material für die Dosiervorrichtung und/oder eine Verbindungsleitung der Dosiervorrichtung, wie zum Beispiel eine Zuführleitung oder eine Abgabeleitung, verwendet. Ist in der abzugebenden Substanz Luft oder ein Gas vorhanden, so kann ein zum Beispiel luftdurchlässiger Zuführschlauch, welcher die Dosiervorrichtung mit dem Vorratsbehälter verbindet und somit der Dosierung vorgeschaltet ist, die Möglichkeit schaffen, dass das in der abzugebenden Substanz enthaltene Gas, welches zusammen mit der Substanz durch diese Leitung geführt wird, entweichen kann, um im Idealfall in der Dosiereinheit keine Gaseinschlüsse mehr in der Substanz zu haben.

Vorzugsweise ist an der Dosiervorrichtung eine Dichtung, wie zum Beispiel eine Dichtungsmanschette, vorgesehen, um den Zufuhr- und Abgabeanschluss der Dosiereinheit abzudichten, wenn dieser Anschluss nicht mit einer Zufuhr- oder Abgabeleitung verbunden ist und zum Beispiel mit der Dosiereinheit von der Verbindung mit der Zufuhrleitung zu der Verbindung mit der Abgabeleitung gedreht wird.

Die Dosiervorrichtung ist vorzugsweise so ausgelegt, dass das Maximalvolumen des veränderbaren Volumens der Dosiereinheit $VOL_{Zylinder,max}$, also zum Beispiel das Volumen eines Zylinders bei einem fast oder vollständig herausgezogenen Kolben, kleiner ist als das 0,1-fache Volumen des Vorratsbehälters oder eines Reservoirs $VOL_{Reservoir}$, in welchem die gesamte dosiert abzugebende Substanz enthalten ist. Bevorzugt ist das maximale Volumen der Dosiereinheit größer als das 2-fache oder auch z.B. 1 0-fache des Volumens einer minimal abzugebenden Dosis $VOL_{Dose,\,min}$. Vorzugsweise ist das maximale Volumen der Dosiereinheit gleich dem minimalen Volumen der abzugebenden Dosis multipliziert mit der Wurzel aus dem Quotienten gebildet aus dem Volumen des Reservoirs und dem minimalen Volumen der minimal abzugebenden Dosis und genügt somit der Formel:

$$VOL_{Zylinder,\,max} = VOL_{Dose,\,min} \times \sqrt{(VOL_{Reservoir}/VOL_{Dose,\,min})}$$

Bevorzugt ist die Dosiervorrichtung so ausgelegt dass:

$$2...10 \times Vol_{Dose,min} < Vol_{Zylinder,max} < 1/10...1/2 \, Vol_{Reservoir}$$

Vorteilhaft ist das Verhältnis des Innendurchmessers einer zylinderförmigen Dosiereinheit zu der Zylinderlänge gleich oder etwa 1:4.

Vorteilhaft wird die Dosiereinheit nach dem Befüllen des veränderbaren Volumens mit der abzugebenden Substanz gedreht, um die Öffnung der Dosiereinheit von der Zufuhrleitung zu entkoppeln und mit der Abgabeleitung zu verbinden. Nicht erfindungsgemäß kann die Dosiereinheit auch verschoben werden.

Erfindungsgemäß kann somit durch eine Dosiervorrichtung unabhängig von der Ausbildung und Größe eines Reservoirs oder eines Vorratsbehälters eine genaue Dosierung der abzugebenden Substanz vorgenommen werden, wobei die Dosiereinheit im Falle der Verwendung einer einzigen Zufuhr- und Abgabeöffnung ein relativ kleines Leck- oder Dichtungsrisiko aufweist.

Die Erfindung wird nachfolgend anhand von Ausführungsbeispielen beschrieben, wobei die beiliegenden Figuren zeigen:

Figuren 1A, B, C      einen prinzipiellen Funktionsablauf beim Dosieren mit der Dosiervorrichtung;

Figur 2      eine Ausführungsform der Dosiervorrichtung;

Figur 3      einen Kreisprozess bei einer Ausführungsform der Erfindung; und

Figur 4          einen Kreisprozess bei einer weiteren Ausführungsform der Erfindung.

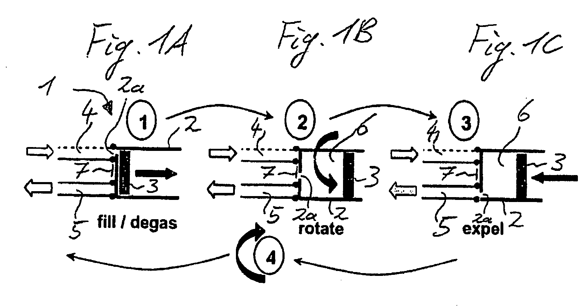

Figur 1A zeigt eine Dosiervorrichtung 1 mit einem Zylinder 2, welcher an einer Stirn- oder Unterseite eine einzige Öffnung 2a aufweist. In der in Figur 1A gezeigten Ausgangslage vor der Befüllung des Zylinders 2 ist die Öffnung 2a mit einer Zuleitung 4 verbunden, welche mit einem nicht gezeigten zum Beispiel unter Druck stehenden Reservoir verbunden ist, das eine dosiert abzugebende Substanz, wie zum Beispiel Insulin, enthält. In dem Zylinder 2 ist ein beweglicher Kolben 3 angeordnet.

Figur 1B zeigt die Dosiervorrichtung 1 gemäß Figur 1A nach dem Aufdosieren der Dosiereinheit 2, 3 durch ein Zurückziehen des Kolbens 3 innerhalb des Zylinders 2, wodurch durch die Zuleitung 4 eine dosiert abzugebende Substanz in das veränderbare Volumen 6 des Zylinders 2 eingebracht wurde. In Figur 1B ist der Zylinder 2 nach dem Abschluss des Befüllvorganges bereits ein Stück um seine Längsachse gedreht worden, wie durch den Pfeil angedeutet, so dass die Öffnung 2a des Zylinders 2 nicht mehr mit der Zuleitung 4 verbunden ist oder in Fluid-Kommunikation mit dieser steht. Die Öffnung 2a ist in dem in Figur 1B gezeigten Zustand in Richtung auf die Ableitung 5 gedreht worden, wobei in dem in Figur 1B gezeigten Zwischenzustand die Öffnung 2a mittels einer an dem Zylinder 2 anliegenden Dichtung 7 verschlossen wird.

Wird der Zylinder 2 so weit gedreht, bis die Öffnung 2a an der Ableitung 5 anliegt, wie in Figur 1C gezeigt, kann durch ein Einschieben des Kolbens 3 in den Zylinder 2 die in dem Volumen 6 enthaltene aufdosierte Substanz durch die Öffnung 2a an die Ableitung 5 abgegeben werden, welche mit einem nicht gezeigten Infusions-Set oder einer Nadel verbunden ist. Nach teilweiser oder vollständiger Abgabe der in dem variablen Volumen 6 des Zylinders 2 enthaltenen Substanz kann der Zylinder 2 zum Beispiel mit vollständig in den Zylinder 2 eingeschobenem Kolben 3 so gedreht werden, dass die Öffnung 2a wieder an der Zuleitung 4 anliegt, wie in Figur 1 durch den Zustand (4) angedeutet, so dass der in Figur 1 gezeigte Zyklus erneut durchlaufen werden kann.

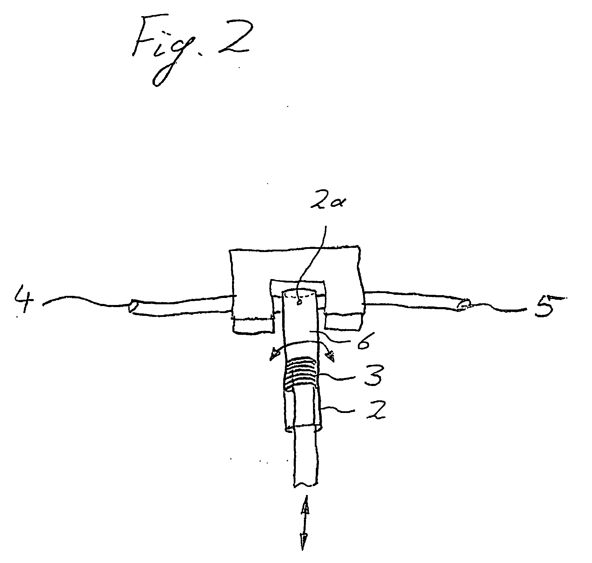

Figur 2 zeigt eine Ausführungsform der erfindungsgemäßen Dosiervorrichtung mit einem aus Kunststoff oder einem z.B. elastischen Material gefertigten drehbaren Zylinder 2, welcher zwischen zwei Leitungen 4, 5 z.B. aus elastischem Material und z.B. auch innerhalb einer Dichthülse mit z.B. zwei Öffnungen angeordnet ist. Durch die in Figur 2 links gezeigte Zuleitung 4 kann das variable Volumen 6 innerhalb des Zylinders 2 durch das Zurückziehen des Kolbens 3 mit einer dosiert abzugebenden Substanz aufgefüllt werden, wenn die an einer seitlichen Oberfläche oder dem Zylindermantel vorgesehene Öffnung 2a in Verbindung mit der Zuleitung 4 ist. Die Öffnung 2a kann ein einfaches Loch in dem Kunststoff-Zylinder 2 sein, welches sich durch die elastische Eigenschaft des Materials des Zylinders 2 verschließt und welches ein Hindurchtreten einer Substanz nur ermöglicht, wenn ein Druck oder Sog auf die Substanz einwirkt, wie zum Beispiel durch das Herausziehen oder Einschieben des Kolbens 3 in den Zylinder 2.

Nach dem Aufdosieren des Zylinders 2, bei welchem eine Substanz über die Zuleitung 4 durch die seitliche Öffnung 2a in den Zylinder 2 gelangt, wird der Zylinder 2 so gedreht, im Ausführungsbeispiel um 180 Grad um seine Längsachse, dass die einzige Öffnung 2a des Zylinders 2 in Verbindung mit der Ableitung 5 steht. In diesem Zustand kann der Kolben 3 wieder in den Zylinder 2 eingeschoben werden, um dadurch die in dem Zylinder 2 enthaltene Substanz zu verdrängen und durch die geöffnete selbst schließende Öffnung 2a des Zylinders 2 an die Ableitung 5 abzugeben, welche mit einem nicht gezeigten Infusionsset verbunden ist.

Anschließend kann der Zylinder 2 wieder zurückgedreht oder weitergedreht werden, bis die seitliche Öffnung 2a des Zylinders 2 wieder in Fluid-Verbindung mit der Zuleitung 4 steht, um erneut eine dosiert abzugebende Substanz durch die Zuleitung 4 aufzunehmen.

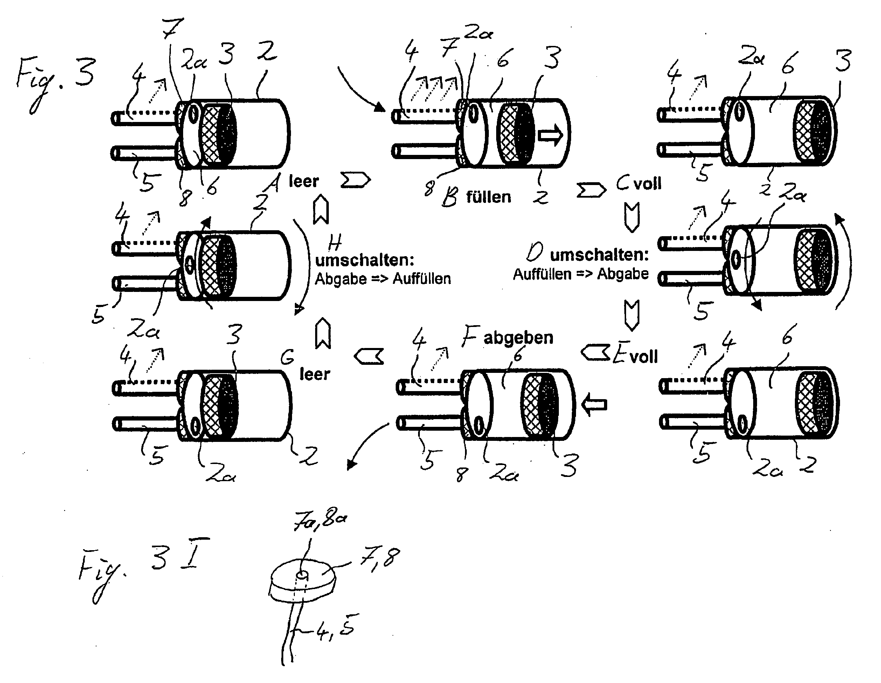

Figur 3 zeigt einen Kreisprozess, welcher einen Funktionsablauf beim Dosieren mit einer Ausführungsform der erfindungsgemäßen Dosiervorrichtung veranschaulicht. Ausgehend von einem mit A bezeichneten Anfangszustand, in welchem der Zylinder 2 leer ist und der Kolben 3 fast vollständig oder vollständig in den Zylinder 2 in Richtung auf die Öffnung 2a an der Zylinderstirnseite eingefahren ist, wird der Zylinder 2 durch die Zuleitung 4, welche luftdurchlässig ausgebildet ist, allmählich gefüllt, wie in Figur 3B gezeigt, wobei der Kolben 3 in der durch den Pfeil gezeigten Richtung innerhalb des Zylinders 2 so bewegt wird, dass sich das veränderbare Volumen 6 vergrößert, wodurch in dem Zylinder 2 ein Unterdruck erzeugt werden kann, wodurch die von einem Reservoir (nicht gezeigt) zuzuführende Substanz durch die Zuleitung 4 angesaugt oder in den Zylinder 2 eingebracht wird, Dabei ist der Zylinder 2 oder die Öffnung 2a so positioniert oder gedreht, dass diese möglichst vor der Öffnung der Zuleitung 4 liegt, so dass eine Fluidverbindung von der Zuleitung 4 zu dem veränderbaren Volumen 6 durch die Öffnung 2a geschaffen wird.

An der Vorderseite bzw. der Verbindungs- oder Kontaktseite der Zuleitung 4 und der Ableitung 5 mit dem Zylinder 2 sind in der gezeigten Ausführungsform Dichtelemente 7 und 8 vorgesehen, welche um die Öffnungen der Zuleitung 4 und der Ableitung 5 herum angeordnet sind und ein Austreten der in diesen Leitungen 4, 5 geführten Substanz oder Flüssigkeit verhindern sollen. Die Dichtungen 7 und 8 können z.B. aus scheibenförmigen oder ringförmigen Elementen bestehen, wie in Figur 3I gezeigt, welche zum Beispiel durch ein elastisches Material gebildet werden, wobei der Zylinder 2 oder eine Stirnseite des Zylinders 2 vorzugsweise leicht über die Dichtelemente 7 und 8 gleiten können.

Wie aus Figur 3C ersichtlich ist, befindet sich der Zylinder 2 im vollständig gefüllten Zustand, wenn der Kolben 3 bis zu einer Maximalposition herausgezogen wurde, wobei dieses Maximalvolumen der Dosiereinheit mit VOL$_{Zylinder,max}$ bezeichnet wird.

Anschließend wird der Zylinder 2 um seine Längsachse gedreht, wie in Figur 3D gezeigt, um von dem Auffüllvorgang des Zylinders 2 zu dem Abgabevorgang umzuschalten. Dabei wird die Drehung des Zylinders 2 so lange durchgeführt, bis die Öffnung 2a an der Abgabeleitung 5 liegt, wie in Figur 3E gezeigt, so dass eine Fluidverbindung zwischen dem veränderbaren Volumen 6 des Zylinders 2 und der Abgabeleitung 5 durch die Öffnung 2a hergestellt wird.

Wird nun der Kolben 3 wieder in den Zylinder 2 eingefahren, wie in Figur 3F gezeigt, so wird die in dem veränderbaren Volumen 6 enthaltene Substanz durch die Öffnung 2a und die Ableitung 5 z.B. an ein Infusionsset abgegeben. Dies kann solange durchgeführt werden, bis der Zylinder 2 vollständig oder fast vollständig entleert ist, wie in Figur 3G gezeigt. Dabei ist der Kolben 3 möglichst bis zu einem vorderen oder Abgabeende des Zylinders 2 in diesen eingefahren.

Anschließend kann, wie in Figur 3H gezeigt, die Dosiereinheit wieder umgeschaltet werden, um nach erfolgtem Abgabevorgang das veränderbare Volumen 6 wieder aufzufüllen. Zur Umschaltung der Dosiervorrichtung wird der Zylinder 2 wieder so gedreht, dass die Öffnung 2a wieder vor der Zuleitung 4 liegt, wie in Figur 3A gezeigt.

Das Hin- und Herdrehen des Zylinders 2, wie in den Figuren 3D und 3H gezeigt, kann sowohl durch eine Drehung in die gleiche Richtung, also z.B. bezogen auf die Längsachse des Zylinders immer im Uhrzeigersinn oder immer gegen den Uhrzeigersinn, erfolgen. Alternativ ist es auch möglich, dass der Zylinder 2 zum Umschalten von einem Auffüllvorgang in den Abgabevorgang, wie in Figur 3D gezeigt, in eine erste Richtung, wie z.B. nach links, gedreht wird, und zum Umschalten von dem Abgabevorgang zum Auffüllvorgang, wie in Figur 3H gezeigt, in die entgegengesetzte Richtung, z.B. nach rechts, gedreht wird, Dies ist insbesondere dann vorteilhaft, wenn mit einem einzigen Motor eine Hin- und Herbewegung des Stopfens 3 realisiert werden soll, wobei der Motor auch noch zur Drehung des Zylinders 2 verwendet wird.

Figur 3I zeigt in perspektivischer Ansicht ein Dichtelement 7, 8 mit der darin angeordneten Durchtrittsöffnung 7a bzw. 8a, an welche sich die Zuleitung 4 in den Zuständen gemäß Figuren 3A bis 3C bzw. die Ableitung 5 in den Zuständen gemäß Figuren 3E bis 3G anschließt.

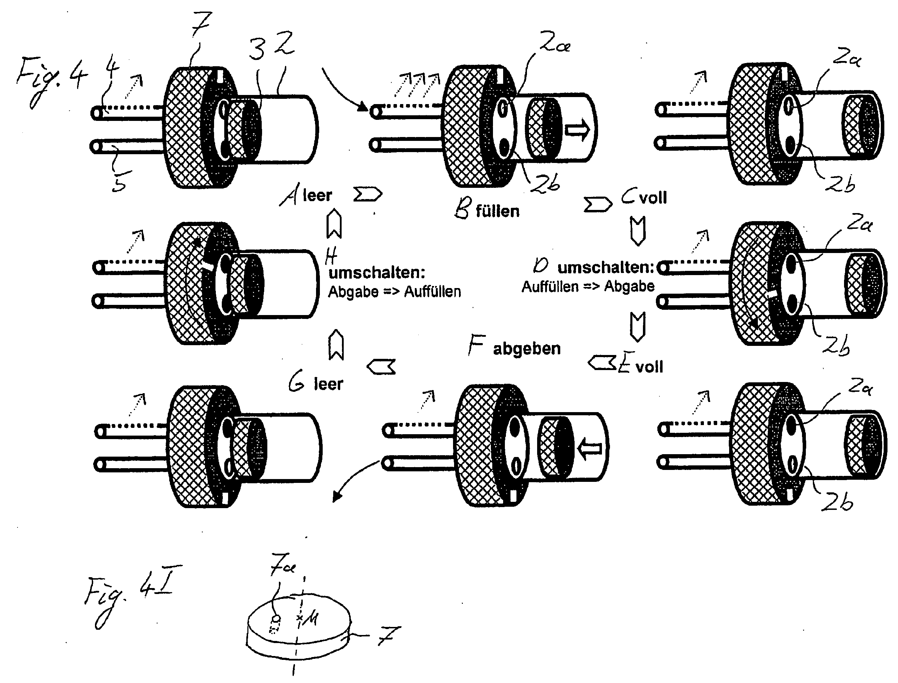

Figur 4 zeigt eine weitere Ausführungsform der Erfindung, wobei der Funktionsablauf ähnlich wie in Figur 3 ist und ein Zylinder 2 an seiner zum Befüllen und Abgeben dienenden Stirnseite Öffnungen 2a und 2b aufweist, welche z.B. bezogen auf einen Mittelpunkt der Stirnfläche des Zylinders 2 einander gegenüberliegend angeordnet sein können. Vor den Öffnungen 2a und 2b des Zylinders 2 ist ein Dichtelement 7 angeordnet, in welchem eine durchgehende Öffnung 7a vorgesehen ist; siehe Fig. 4I. Das Dichtelement 7 ist relativ zu dem Zylinder 2 und der Zuführleitung 4, sowie der Abführleitung 5, drehbar oder verschiebbar. Vorzugsweise befinden sich der Zylinder 2, die Zuführleitung 4 und die Abführleitung 5 in einem definierten oder festen Lageverhältnis. Durch die Drehung des Dichtelementes 7 mit der darin angeordneten Öffnung 7a können die Öffnungen 2a und 2b des Zylinders 2 entweder beide verschlossen werden, wie in den Umschaltvorgängen in den Figuren 4D und 4H gezeigt, oder alternierend geöffnet werden.

Während des Auffüllvorganges, wie in dem Ablauf der Figuren 4A, 4B und 4C gezeigt, befindet sich die Öffnung 7a des Dichtelementes 7 in einer Position, in welcher eine Fluidverbindung zwischen der Zuführleitung 4 und dem veränderbaren Volumen 6 hergestellt werden kann. Zum Umschalten zwischen dem Auffüllvorgang und dem Abgabevorgang wird das Dichtelement 7 um seine Mittelachse M gedreht, wie in Figur 4D gezeigt, bis die Öffnung 7a des Dichtelementes 7 so angeordnet ist, dass die Zuführöffnung 2a des Zylinders 2 verschlossen ist und die Abgabeöffnung 2b des Zylinders mit der Abgabeleitung 5 fluidisch gekoppelt ist, um den in den Figuren 4E, 4F und 4G gezeigten Abgabevorgang durchzuführen.

Anschließend kann von dem Abgabevorgang wieder in den Auffüllvorgang umgeschaltet werden, wie in Figur 4H gezeigt, wobei das Dichtelement 7 wieder so gedreht wird, dass die Öffnung 7a des Dichtelementes 7 in der in Figur 4A gezeigten Position ist.

Figur 4I zeigt in perspektivischer Ansicht das Dichtelement 7 mit einem von dem Mittelpunkt bzw. Drehpunkt M des Dichtelementes 7 in radiale Richtung versetzten Durchgang oder Durchleitungsstück 7a.

**Patentansprüche**

1. Infusionssystem mit einer Dosiervorrichtung für das Infusionssystem mit einer Dosiereinheit (2, 3) mit einem in Zwischenschritten oder stufenweise veränderbaren Volumen (6) und mit einem einzigen Zufuhr- und Abgabeanschluss (2a) oder mit alternierend schließbaren oder öffenbaren Anschlüssen (2a, 2b), durch welche das veränderbare Volumen (6) der Dosiereinheit (2, 3 ) mit einer Substanz gefüllt und/oder eine in dem veränderbaren Volumen (6) enthaltene Substanz abgegeben werden kann, wobei die Dosiereinheit (2, 3) ein Zylinder (2) mit einem in den Zylinder (2) durch einen Motor einschiebbaren Kolben (3) ist und mit einem Infusions-Set, welches mit dem einzigen Zufuhr- und Abgabeanschluss (2a) durch Drehung des Zylinders um seine Längsachse, oder mit einem der alternierend schließbaren oder öffenbaren Anschlüsse (2a, 2b) durch Drehung des Zylinders um seine Längsachse oder durch Drehung oder Verschiebung eines Dichtelements (7) mit einer darin angeordneten Öffnung (7a) mittels des oder eines anderen Motors verbindbar ist.

2. Infusionssystem nach Anspruch 1, wobei der Zufuhr- und Abgabeanschluss (2a, 2b) aus mindestens einer Öffnung der Dosiereinheit (2, 3) besteht.

3. Infusionssystem nach einem der vorhergehenden Ansprüche mit einer Zufuhrleitung (4) und einer Abgabeleitung (5), welche abwechselnd oder alternierend mit dem Zufuhr- und Abgabeanschluss (2a, 2b) der Dosiereinheit (2, 3) verbunden werden können.

4. Infusionssystem nach einem der vorhergehenden Ansprüche mit mindestens einem Ventil in der Dosiereinheit (2, 3) oder in oder an dem Zufuhr- und Abgabeanschluss (2a, 2b) und/oder an der Zufuhrleitung (4) und/oder an der Abgabeleitung (5).

5. Infusionssystem nach einem der zwei vorhergehenden Ansprüche mit mindestens einem Motor, welcher die Zufuhrleitung (4) und/oder Abgabeleitung (5) verschieben kann.

6. Infusionssystem nach einem der vorhergehenden Ansprüche mit einem Sensor oder Detektor zum Detektieren eines Lecks, einer Okklusion, eines Drucks, einer Kraft oder von Blasen.

7. Infusionssystem nach einem der vorhergehenden Ansprüche, wobei die Dosiereinheit (2, 3) und/oder die Zufuhrleitung (4) und/oder die Abgabeleitung (5) aus einem luftdurchlässigen Material sind.

8. Infusionssystem nach einem der vorhergehenden Ansprüche mit einer Dichtung (7), um den Zufuhr- und Abgabeanschluss (2a, 2b) in einem Betriebszustand zwischen der Aufnahme und der Abgabe einer Substanz abzudichten.

9. System zur Abgabe einer Substanz mit einem Infusionssystem nach einem der vorhergehenden Ansprüche und einem Vorratsbehälter, welcher mit einer Zufuhrleitung (4) verbunden ist.

10. System nach dem vorhergehenden Anspruch, wobei der Vorratsbehälter so mit einer Energie, Kraft oder einem Druck beaufschlagt ist, dass eine in dem Behälter enthaltene Substanz durch einen Abgabeanschluss des Behälters an die Zufuhrleitung (4) abgegeben werden kann oder wird.

**Claims**

1. An infusion system comprising a dosing device for the infusion system comprising a dosing unit (2, 3) which exhibits a volume (6) which is variable in intermediate steps or increments and comprises a single supplying and dispensing port (2a) or ports (2a, 2b) which can be alternately closed or opened, through which the variable volume (6) of the dosing unit (2, 3) can be filled with a substance and/or a substance contained in the variable volume (6) can be dispensed, wherein the dosing unit (2, 3) is a cylinder (2) comprising a piston (3) which can be slid into the cylinder (2) by a motor and comprising an infusion set which can be connected by means of the motor or by means of another motor to the single supplying and dispensing port (2a) by rotating the cylinder about its longitudinal axis, or to one of the ports (2a, 2b) which can be alternately closed or opened by rotating the cylinder about its longitudinal axis or rotating or shifting a sealing element (7) with an opening (7a) arranged in it.

2. The infusion system according to claim 1, wherein the supplying and dispensing port (2a, 2b) consists of at least one opening of the dosing unit (2, 3).

3. The infusion system according to any one of the preceding claims, comprising a supplying conduit (4) and a dispensing conduit (5) which can be alternately connected to the supplying and dispensing port (2a, 2b) of the dosing unit (2, 3).

4. The infusion system according to any one of the preceding claims, comprising at least one valve in the dosing unit (2, 3) or in or on the supplying and dispensing port (2a, 2b) and/or on the supplying conduit (4) and/or on the dispensing conduit (5).

5. The infusion system according to any one of the preceding two claims, comprising at least one motor which can shift the supplying conduit (4) and/or dispensing conduit (5).

**6.** The infusion system according to any one of the preceding claims, comprising a sensor or detector for detecting a leakage, an occlusion, a pressure, a force or bubbles.

**7.** The infusion system according to any one of the preceding claims, wherein the dosing unit (2, 3) and/or the supplying conduit (4) and/or the dispensing conduit (5) is made from a material which is permeable to air.

**8.** The infusion system according to any one of the preceding claims, comprising a seal (7) for sealing off the supplying and dispensing port (2a, 2b) in an operational state between receiving and dispensing a substance.

**9.** A system for dispensing a substance, comprising an infusion system according to any one of the preceding claims and a repository container which is connected to a supplying conduit (4).

**10.** The system according to the preceding claim, wherein the repository container is charged with energy, a force or a pressure in such a way that a substance contained in the container can be dispensed or is dispensed to the supplying conduit (4) through a dispensing port of the container.


**Revendications**

**1.** Système de perfusion comportant un dispositif de dosage pour le système de perfusion, doté d'une unité de dosage (2, 3) présentant un volume (6) pouvant être modifié par étapes intermédiaires ou par paliers et un raccord d'alimentation et de distribution unique (2a) ou des raccords (2a, 2b) pouvant être fermés ou ouverts en alternance par lesquels le volume modifiable (6) de l'unité de dosage (2, 3) peut être rempli d'une substance et/ou une substance contenue dans le volume (6) modifiable peut être distribuée, dans lequel l'unité de dosage (2, 3) est un cylindre (2) avec un piston (3) pouvant être poussé dans le cylindre (2) par un moteur et comportant un ensemble de perfusion qui peut être relié avec le raccord d'alimentation et de distribution unique (2a) par rotation du cylindre autour de son axe longitudinal, ou avec le raccord (2a, 2b) pouvant être fermé ou ouvert en alternance par rotation du cylindre autour de son axe longitudinal, ou par rotation ou coulissement d'un élément d'étanchéité (7) avec une ouverture (7a) disposée dans celui-ci, au moyen du moteur ou d'un autre moteur.

**2.** Système de perfusion selon la revendication 1, dans lequel le raccord d'alimentation et de distribution (2a, 2b) est constitué d'au moins une ouverture de l'unité de dosage (2, 3).

**3.** Système de perfusion selon l'une des revendications précédentes, comportant une conduite d'alimentation (4) et une conduite de distribution (5) qui peuvent être reliées alternativement ou en variante avec un raccord d'alimentation et de distribution (2a, 2b) de l'unité de dosage (2, 3).

**4.** Système de perfusion selon l'une des revendications précédentes, comportant au moins une soupape dans l'unité de dosage (2, 3) ou dans le raccord d'alimentation et de distribution ou au niveau de celui-ci (2a, 2b) et/ou au niveau de la conduite d'alimentation (4) et/ou de la conduite de distribution (5).

**5.** Système de perfusion selon l'une des deux revendications précédentes, comportant au moins un moteur qui peut coulisser la conduite d'alimentation (4) et/ou la conduite de distribution (5).

**6.** Système de perfusion selon l'une des revendications précédentes, comportant un capteur ou un détecteur pour détecter une fuite, une occlusion, une pression, une force ou des bulles.

**7.** Système de perfusion selon l'une des revendications précédentes, dans lequel l'unité de dosage (2, 3) et/ou la conduite d'alimentation (4) et/ou la conduite de distribution (5) sont en un matériau perméable à l'air.

**8.** Système de perfusion selon l'une des revendications précédentes, présentant un joint (7) pour étanchéifier le raccord d'alimentation et le raccord de distribution (2a, 2b) à l'état de fonctionnement, entre la réception et l'administration d'une substance.

**9.** Système d'administration d'une substance avec un système de perfusion selon l'une des revendications précédentes et un réservoir qui est relié à la conduite d'alimentation (4).

**10.** Système selon la revendication précédente, dans lequel le réservoir est alimenté avec une énergie, une force ou une pression telle qu'une substance contenue dans le réservoir soit ou puisse être administrée par un raccord de distribution du réservoir dans la conduite d'alimentation (4).

Fig. 1A    Fig. 1B    Fig. 1C

fill / degas    rotate    expel

Fig. 2

Fig. 3

Fig. 3 I

Fig. 4

Fig. 4I

**EP 1 970 677 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CH 688224 A5
- US 6010485 A
- US 6749587 B2
- US 4643723 A
- WO 9304714 A
- EP 0600948 B1
- US 20040069044 A1
- US 5207666 A
- US 20050159708 A1
- EP 1633417 B1
- US 5807321 A
- WO 2007000064 A1
- EP 0980690 A2
- EP 1754505 A1